(19) Europäisches Patentamt
European Patent Office
Office européen des brevets

(11) Numéro de publication: **0 506 549 B1**

(12) **FASCICULE DE BREVET EUROPEEN**

(45) Date de publication du fascicule du brevet: **18.10.95**

(51) Int. Cl.6: **A61K 7/13**

(21) Numéro de dépôt: **92400799.0**

(22) Date de dépôt: **25.03.92**

(54) **Composition tinctoriale pour fibres kératiniques, contenant des précurseurs de colorants d'oxydation et des hydroxybenzofurannes utilisés comme coupleurs, et procédé de teinture mettant en oeuvre ces compositions.**

(30) Priorité: **28.03.91 FR 9103806**

(43) Date de publication de la demande:
**30.09.92 Bulletin 92/40**

(45) Mention de la délivrance du brevet:
**18.10.95 Bulletin 95/42**

(84) Etats contractants désignés:
**AT BE CH DE DK ES FR GB GR IT LI NL PT SE**

(56) Documents cités:
**EP-A- 0 023 080**
**EP-A- 0 026 474**
**DE-A- 2 410 634**
**DE-A- 2 719 424**

**CHEMICAL ABSTRACTS, vol. 79, no. 23, 10 Décembre 1973, Columbus, Ohio, US; abstract no. 136921O, LOIC RENE ET AL: 'Benzofurans. LIV. New access to hydroxybenzofurans' page 361 ;colonne 2 ;**

(73) Titulaire: **L'OREAL**
**14, rue Royale**
**F-75008 Paris (FR)**

(72) Inventeur: **Tuloup, Rémy**
**Le Bourg**
**F-35190 Miniac-sous-Becherel (FR)**
Inventeur: **Blaise, Christian**
**24-81 Allée Jean Masaryk**
**F-93270 Sevran (FR)**
Inventeur: **Junino, Alex**
**16, rue Docteur Bergonié**
**F-93180 Livry-Gargan (FR)**

(74) Mandataire: **Casalonga, Axel et al**
**BUREAU D.A. CASALONGA - JOSSE**
**Morassistrasse 8**
**D-80469 München (DE)**

**EP 0 506 549 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention est relative à de nouvelles compositions tinctoriales pour fibres kératiniques et en particulier pour cheveux humains, contenant des précurseurs de colorants d'oxydation et des hydroxybenzofuranne utilisés comme coupleurs et un procédé de teinture mettant en oeuvre de telles compositions.

Il est connu de teindre les fibres kératiniques et en particulier les cheveux humains, avec des compositions tinctoriales contenant des précurseurs de colorants d'oxydation et en particulier des ortho ou para phénylène diamines, des ortho- ou para-aminophénols, appelés généralement "bases d'oxydation".

On sait également qu'on peut faire varier les nuances obtenues avec ces bases d'oxydation en les associant à des coupleurs encore appelés modificateurs de coloration, choisis notamment parmi des métadiamines aromatiques, des méta-aminophénols et des métadiphénols.

On recherche, dans le domaine de la teinture capillaire, des précurseurs de colorants d'oxydation ou des coupleurs permettant de conférer aux cheveux, en milieu alcalin ou acide oxydant, une coloration ayant une résistance satisfaisante à la lumière, aux lavages, aux intempéries et à la transpiration.

La demanderesse vient de découvrir, ce qui fait l'objet de l'invention, que l'utilisation de 4-, 5-, 6- ou 7-hydroxybenzofurannes en tant que coupleurs, avec des précurseurs de colorants d'oxydation, permettait d'obtenir après application sur les fibres kératiniques et en particulier les cheveux, des teintures présentant des résistances à la lumière, aux lavages, aux intempéries et à la transpiration, particulièrement remarquables, notamment lorsqu'ils sont utilisés avec la p-phénylènediamine et ses dérivés.

Un objet de l'invention est donc constitué par des compositions tinctoriales d'oxydation, destinées à être utilisées pour la teinture des fibres kératiniques, contenant au moins un précurseur de colorant d'oxydation de type para et/ou ortho avec certains hydroxybenzofurannes définis ci-après.

Un autre objet de l'invention est constitué par le procédé de coloration des fibres kératiniques, en particulier des cheveux humains, mettant en oeuvre de telles compositions.

D'autres objets de l'invention apparaîtront à la lecture de la description et des exemples qui suivent.

La composition tinctoriale d'oxydation, conforme à l'invention, destinée à être utilisée pour la teinture des fibres kératiniques et en particulier des cheveux humains, est essentiellement caractérisée par le fait qu'elle contient dans un milieu approprié pour la teinture, au moins un précurseur de colorant d'oxydation para et/ou ortho et au moins un coupleur hétérocyclique, répondant à la formule (I) :

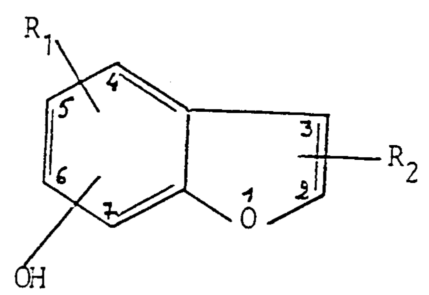

(I)

dans laquelle $R_1$ et $R_2$, identiques ou différents, représentent un atome d'hydrogène ou un radical alkyle en $C_1$-$C_6$. $R_1$ et $R_2$ peuvent occuper n'importe quelle position du noyau benzofuranne. Cependant, lorsque $R_1$ et $R_2$ désignent tous deux un radical alkyle, ils n'occupent pas à la fois les positions 2 et 3. Le groupement OH peut occuper l'une des positions 4, 5, 6 ou 7.

Parmi les significations préférées du radical alkyle, il faut citer les suivantes : méthyle, éthyle, propyle, isopropyle, butyle, isobutyle, tertiobutyle, pentyle et hexyle.

Ces composés et leur procédé de préparation sont connus.

Notamment, l'article de Loïc RENE et Ren ROYER dans le Bulletin de la Société Chimique de France, 1973, 2355-2356, décrit la préparation des hydroxybenzofurannes.

L'ouvrage "Heterocyclic Compounds", Volume 29, Benzofurannes (1974) par MUSTAFA, Editeur : WILEY INTERSCIENCE, décrit la préparation des hydroxybenzofurannes et hydroxybenzofurannes substitués.

Cependant, leur utilisation dans une composition tinctoriale en tant que coupleur en association avec un ou plusieurs précurseurs de colorant d'oxydation, est nouvelle.

Parmi les hydroxybenzofurannes substitués de formule (I), il faut citer plus particulièrement les suivants :

- le 2-méthyl 6-hydroxybenzofuranne,
- le 3-méthyl 6-hydroxybenzofuranne,
- le 2,4-diméthyl 6-hydroxybenzofuranne,
- le 3-n-propyl 6-hydroxybenzofuranne,
- le 2-éthyl 5-hydroxybenzofuranne,
- le 2-méthyl 5-hydroxybenzofuranne,
- le 3-méthyl 5-hydroxybenzofuranne,
- le 3-isobutyl 5-hydroxybenzofuranne,
- le 3-éthyl 5-hydroxybenzofuranne,
- le 2,6-diméthyl 5-hydroxybenzofuranne,
- le 3,6-diméthyl 5-hydroxybenzofuranne,
- le 6,7-diméthyl 5-hydroxybenzofuranne,
- le 3-n-propyl 5-hydroxybenzofuranne,
- le 3-méthyl 4-n-propyl 5-hydroxybenzofuranne,
- le 2-hexyl 5-hydroxybenzofuranne,
- le 2-n-propyl 5-hydroxybenzofuranne,
- le 4-tertiobutyl 5-hydroxybenzofuranne,
- le 6-tertiobutyl 5-hydroxybenzofuranne,
- le 4-méthyl 5-hydroxybenzofuranne,
- le 3-isobutyl 5-hydroxybenzofuranne,
- le 3-méthyl 5-n-propyl 4-hydroxybenzofuranne,
- le 2-éthyl 4-hydroxybenzofuranne,
- le 2-méthyl 6-pentyl 4-hydroxybenzofuranne,
- le 6-pentyl 4-hydroxybenzofuranne,
- le 3,5-diméthyl 4-hydroxybenzofuranne,
- le 3,7-diméthyl 4-hydroxybenzofuranne,
- le 2,6-di-tertiobutyl 4-hydroxybenzofuranne,
- le 2-méthyl 4-hydroxybenzofuranne,
- le 3-méthyl 4-hydroxybenzofuranne,
- le 2-méthyl 7-éthyl 4-hydroxybenzofuranne,
- le 2,7-diméthyl 4-hydroxybenzofuranne,
- le 2-isopropyl 4-hydroxybenzofuranne,
- le 3-éthyl 4-hydroxybenzofuranne,
- le 3-méthyl 7-tertiobutyl 4-hydroxybenzofuranne,
- le 3-méthyl 5-tertiobutyl 4-hydroxybenzofuranne,
- le 2,6-diméthyl 4-hydroxybenzofuranne,
- le 3-isopropyl 4-hydroxybenzofuranne,
- le 3-n-propyl 4-hydroxybenzofuranne,
- le 3-méthyl 7-n-propyl 4-hydroxybenzofuranne,
- le 3-méthyl 6-n-propyl 7-hydroxybenzofuranne,
- le 3-méthyl 7-hydroxybenzofuranne,
- le 2-éthyl 4-méthyl 7-hydroxybenzofuranne,
- le 2-éthyl 5-méthyl 7-hydroxybenzofuranne.

Les précurseurs de colorants d'oxydation de type para ou ortho sont des composés qui ne sont pas des colorants en eux-mêmes, mais qui forment un colorant par un processus de condensation oxydative, soit sur eux-mêmes, soit en présence d'un coupleur ou modificateur.

Ces composés comportent des groupements fonctionnels, soit deux amino, soit un amino et un hydroxy, en position para ou ortho, l'un par rapport à l'autre.

Les précurseurs de colorants d'oxydation de type para sont en particulier choisis parmi les paraphénylènediamines, les para-amino phénols, les précurseurs hétérocycliques para, comme la 2,5-diaminopyridine, la 2-hydroxy 5-aminopyridine, la tétraaminopyrimidine, et les bases dites "doubles".

A titre de paraphénylènediamines, on peut citer les composés répondant à la formule (II) ci-après :

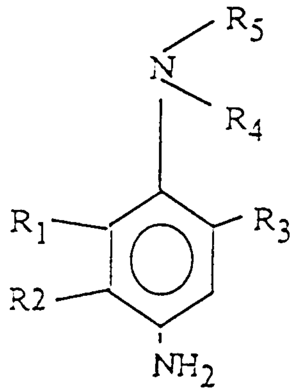

(II)

dans laquelle $R_1$, $R_2$ et $R_3$, identiques ou différents, représentent un atome d'hydrogène ou d'halogène, un radical alkyle ayant 1 à 4 atomes de carbone, un radical alcoxy ayant 1 à 4 atomes de carbone, $R_4$ et $R_5$, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, pipéridinoalkyle, morpholinoalkyle, phényle éventuellement substitué en para par un groupe amino, ces groupes alkyle ou alcoxy ayant de 1 à 4 atomes de carbone, ou bien $R_4$ et $R_5$ forment, conjointement avec l'atome d'azote auquel ils sont liés, un hétérocycle pipéridino ou morpholino, sous réserve que $R_1$ ou $R_3$ représente un atome d'hydrogène lorsque $R_4$ et $R_5$ ne représentent pas un atome d'hydrogène, ainsi que les sels de ces composés.

Parmi les composés de formule (II), on peut citer la p-phénylène diamine, la p-toluylènediamine, la méthoxyparaphénylènediamine, la chloroparaphénylènediamine, la 2,6-diméthylparaphénylènediamine, la 2,6-diéthylparaphénylènediamine, la 2,5-diméthylparaphénylène diamine, la 2-méthyl 5-méthoxyparaphénylènediamine, la 2,6-diméthyl 5-méthoxyparaphénylènediamine, la N,N-diméthylparaphénylène diamine, la N,N-diéthylparaphénylènediamine, la N,N-dipropylparaphénylènediamine, la 3-méthyl 4-amino N,N-diéthylaniline, la N,N-di(β-hydroxyéthyl)paraphénylènediamine, la 3-méthyl 4-amino N,N-di(β-hydroxyéthyl)aniline, la 3-chloro 4-amino N,N-di-(β-hydroxyéthyl) aniline, la 4-amino N,N-(éthyl, carbamylméthyl)aniline, la 3-méthyl 4-amino N,N-(éthyl, carbamylméthyl)aniline, la 4-amino N,N-(éthyl, β-pipéridinoéthyl)aniline, la 3-méthyl 4-amino N,N-(éthyl, β-pipéridinoéthyl)aniline, la 4-amino N,N-(éthyl, β-morpholinoéthyl)aniline, la 3-méthyl 4-amino N,N-(éthyl, β-morpholinoéthyl)aniline, la 4-amino N,N-(éthyl,β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-(éthyl,β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3-méthyl 4-amino N,N-(éthyl,β-sulfoéthyl)aniline, la N-[(4'-amino)phényl]-morpholine, la N-[(4'-amino)phényl]pipéridine, la 2-hydroxyéthylparaphénylènediamine, la fluoroparaphénylènediamine, la carboxyparaphénylènediamine, la sulfoparaphénylènediamine, la 2-isopropylparaphénylènediamine, la 2-n-propylparaphénylènediamine, l'hydroxy-2-n-propylparaphénylène diamine, la 2-hydroxyméthylparaphénylènediamine, la N,N-diméthyl 3-méthylparaphénylènediamine, la N,N-(éthyl,β-hydroxyéthyl)-paraphénylènediamine, la N-(dihydroxypropyl)paraphénylènediamine, la N-4'-aminophénylparaphénylènediamine, la N-phénylparaphénylène diamine.

Ces précurseurs de colorants d'oxydation de type para peuvent être introduits dans la composition tinctoriale, soit sous forme de base libre, soit sous forme de sels, tels que chlorhydrate, bromhydrate ou sulfate.

Parmi les p-aminophénols, on peut citer le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-(β-hydroxyéthyl)-4-aminophénol, le 2-méthoxy 4-aminophénol, le 3-méthoxy 4-aminophénol, le 2,5-diméthyl 4-aminophénol, le 2-méthoxyméthyl 4-amino phénol, le 2-aminométhyl 4-aminophénol et le 2-β-hydroxyéthylaminométhyl 4-aminophénol.

Les précurseurs de colorants d'oxydation de type ortho sont choisis parmi les orthoaminophénols, comme le 1-amino 2-hydroxybenzène, le 6-méthyl 1-hydroxy 2-aminobenzène, le 4-méthyl 1-amino 2-hydroxybenzène, les orthophénylènediamines.

Les bases dites doubles sont des bis-phénylènealkylènediamines, répondant à la formule :

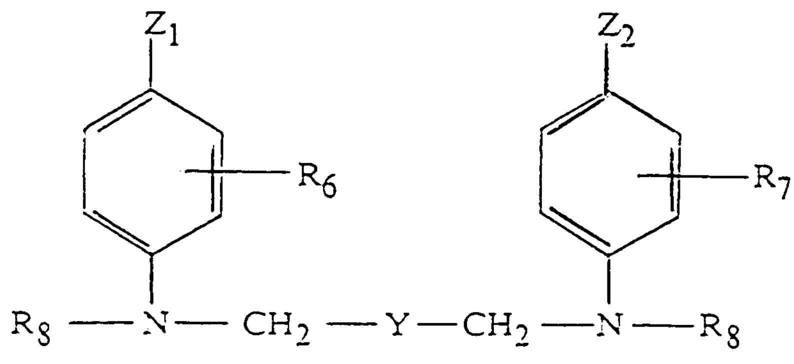

(III)

dans laquelle :

$Z_1$ et $Z_2$, identiques ou différents, représentent des groupements hydroxyle ou $NHR_9$, où $R_9$ désigne un atome d'hydrogène ou un radical alkyle inférieur;

$R_6$ et $R_7$, identiques ou différents, représentent des atomes d'hydrogène, des atomes d'halogène ou encore des groupements alkyle;

$R_8$ représente un atome d'hydrogène, un groupe alkyle, hydroxyalkyle ou aminoalkyle, dont le reste amino peut être substitué par un ou deux groupements alkyle;

Y représente un radical choisi parmi les radicaux suivants :

$-(CH_2)_n-$, $(CH_2)_m-O-(CH_2)_m$,
$-(CH_2)_m-CHOH-(CH_2)_m$,

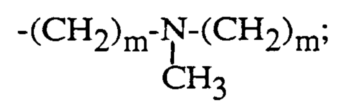

n est un nombre entier compris entre 0 et 8 et m un nombre entier compris entre 0 et 4. Ces bases dite doubles pouvant se présenter également sous forme de ses sels d'addition avec des acides.

Les radicaux alkyle ou alcoxy ci-dessus indiqués désignent de préférence un groupement ayant 1 à 4 atomes de carbone et de préférence méthyle, éthyle, propyle, méthoxy, éthoxy.

Parmi les composés de formule (III), on peut citer le N,N′-bis-(β-hydroxyéthyl)N,N′-bis-(4′-aminophényl)-1,3-diamino 2-propanol, la N,N′-bis-(β-hydroxyéthyl)N,N′-bis-(4′-aminophényl)éthylènediamine, la N,N′-bis-(4-aminophényl)tétraméthylènediamine, la N,N′-bis-(β-hydroxyéthyl)N,N′-bis-(4-aminophényl)-tétraméthylènediamine, la N,N′-bis-(4-méthylaminophényl)tétraméthylènediamine, la N,N′-bis-(ethyl)N,N′-bis-(4′-amino3′-méthylphényl)éthylènediamine.

Les compositions tinctoriales peuvent également contenir en plus du coupleur hétérocyclique de la famille des hydroxybenzofurannes de formule (I) définie ci-dessus, d'autres coupleurs connus en eux-mêmes, tels que les métadiphénols, les métaaminophénols, les métaphénylène diamines, les métaacylaminophénols, les métauréidophénols, les métacarbalcoxyaminophénols, l'α-naphtol, les coupleurs possédant un groupement méthylène actif, tels que les composés β-cétoniques, les pyrazolones, les coupleurs hétérocycliques et le 4-, 6-, ou 7-hydroxyindole.

Parmi ces coupleurs, on peut plus particulièrement citer le 2,4-dihydroxyphénoxyéthanol, le 2,4-dihydroxyanisole, le métaaminophénol, le monométhyléther de résorcine, le 2-méthyl 5-N-(β-hydroxy éthyl)-aminophénol, le 2-méthyl 5-N-(β-mésylaminoéthyl)aminophénol, la 6-hydroxybenzomorpholine, le 2,4-diaminoanisole, le 2,4-diamino phénoxyéthanol, la 6-aminobenzomorpholine, le [2-N-(β-hydroxyéthyl) amino 4-amino]-phénoxyéthanol, le 2-amino 4-N-(β-hydroxyéthyl) amino anisole, le (2,4-diamino)phényl-β-γ-dihydroxypropyléther, la 2,4-diaminophénoxyéthylamine, le 1,3-diméthoxy 2,4-diaminobenzène, le 2-méthyl 5-aminophénol, le 2,6-diméthyl 3-aminophénol, le 1-amino 3,4-méthylène dioxybenzène, le 1-hydroxy 3,4-méthylènedioxybenzène, le 2-chloro 6-méthyl 3-aminophénol, le 2-méthyl 3-aminophénol, le 2-chlororésorcinol, le résorcinol, la 6-méthoxy 3-hydroxyéthylamino aniline, le 1-éthoxy 2-bis-(β-hydroxyéthyl)amino 4-aminobenzène, le 3-diéthylaminophénol, le 1,3-dihydroxy 2-méthylbenzène, le 1-hydroxy 2,4-dichloro 3-aminobenzène, le 4,6-hydroxyéthoxy 1,3-diaminobenzène, le 4-méthyl 6-éthoxy 1,3-diaminobenzène, le 4-chloro 6-méthyl 3-aminophénol, le 6-chloro 3-trifluoroéthylamino phénol.

On peut rajouter à ces compositions, comme cela est bien connu dans l'état de la technique, notamment en vue de nuancer ou d'enrichir en reflets les colorations apportées par les précurseurs de colorants d'oxydation et le coupleur de formule (I), des colorants directs, tels que des colorants azoïques, anthraquinoniques ou les dérivés nitrés de la série benzénique.

L'ensemble des précurseurs de colorants par oxydation de type para et/ou ortho, ainsi que les coupleurs utilisés dans les compositions tinctoriales conformes à l'invention, représente de préférence de 0,3 à 7% en poids par rapport au poids de ladite compositon. La concentration en hydroxybenzofurannes de formule (I) est de 0,05 à 3,5% en poids du poids total de la composition.

Le milieu approprié pour la teinture est constitué généralement par un milieu aqueux et son pH peut varier entre 4 et 11.

Les compositions tinctoriales conformes à l'invention contiennent également, dans leur forme de réalisation préférée, des agents tensioactifs anioniques, cationiques, non-ioniques, amphotères ou leurs mélanges.

Ces agents tensio-actifs sont présents dans les compositions conformes à l'invention dans des proportions comprises entre 0,5 et 55% en poids, et de préférence entre 2 et 50% en poids par rapport au poids total de la composition.

Ces compositions peuvent également contenir des solvants organiques pour solubiliser les composés qui ne seraient pas suffisamment solubles dans l'eau. Parmi ces solvants, on peut citer à titre d'exemple, les alcanols inférieurs en $C_1$-$C_4$, tels que l'éthanol et l'isopropanol; le glycérol; les glycols ou éthers de glycols, comme le 2-butoxyéthanol, l'éthylèneglycol, le propylèneglycol, le monoéthyl éther et le monométhyléther du diéthylèneglycol, ainsi que les alcools aromatiques comme l'alcool benzylique ou le phénoxyéthanol, les produits analogues et leurs mélanges.

Les solvants sont présents de préférence dans des proportions comprises entre 1 et 40% en poids, et en particulier entre 5 et 30% en poids par rapport au poids total de la composition.

Les agents épaississants que l'on peut ajouter dans les compositions conformes à l'invention peuvent être choisis parmi l'alginate de sodium, la gomme arabique, les dérivés de cellulose, les polymères d'acide acrylique, la gomme de xanthane, les scléroglucanes. On peut également utiliser des agents épaississants minéraux, tels que la bentonite.

Ces agents épaississants sont présents de préférence dans des proportions comprises entre 0,1 et 5%, et en particulier entre 0,2 et 3% en poids par rapport au poids total de la composition.

Les agents antioxydants qui peuvent être présents dans les compositions sont choisis en particulier parmi le sulfite de sodium, l'acide thioglycolique, le bisulfite de sodium, l'acide déhydroascorbique, l'hydroquinone et l'acide homogentisique. Ces agents antioxydants sont présents dans la composition dans des proportions comprises entre 0,05 et 1,5% en poids par rapport au poids total de la composition.

Ces compositions peuvent également contenir d'autres adjuvants cosmétiquement acceptables, tels que par exemple des agents de pénétration, des agents séquestrants, des parfums, des tampons, des agents alcalins, des propulseurs.

Les compositions conformes à l'invention peuvent se présenter sous des formes diverses, telles que sous forme de liquides, de crèmes, de gels ou sous toute autre forme appropriée pour réaliser une teinture des fibres kératiniques et notamment des cheveux humains. Ces compositions peuvent être conditionnées en flacons aérosols en présence d'un agent propulseur.

Les compositions tinctoriales conformes à l'invention contenant un précurseur de colorant par oxydation du type para ou du type ortho ou leur mélange et un ou plusieurs hydroxybenzofurannes de formule (I) jouant le rôle de coupleur, sont utilisées dans les procédés de teinture des fibres kératiniques et en particulier des cheveux humains, suivant un procédé mettant en oeuvre la révélation par un agent oxydant.

Conformément à ce procédé on mélange, au moment de l'emploi, la composition tinctoriale décrite ci-dessus avec une solution oxydante en une quantité suffisante pour pouvoir développer une coloration, puis on applique le mélange obtenu sur les fibres kératiniques et en particulier, les cheveux humains.

Le pH de la composition appliquée sur les cheveux varie entre 3,5 et 10.

La solution oxydante contient à titre d'agent oxydant, l'eau oxygénée, le peroxyde d'urée, des persels, tels que le persulfate d'ammonium ou des bromates de métaux alcalins. On utilise de préférence une solution d'eau oxygénée à 20 volumes (6% en poids). Le mélange obtenu est appliqué sur les cheveux et on laisse poser pendant 10 à 40 minutes, de préférence 15 à 30 minutes, après quoi on rince les cheveux, on les lave au shampooing, on les rince à nouveau et on les sèche.

Le coupleur hétérocyclique de la famille des hydroxybenzofurannes de formule (I) définie ci-dessus, peut également être mis en oeuvre dans un procédé à plusieurs étapes, consistant dans l'une des étapes,

à appliquer sur les fibres kératiniques une composition tinctoriale contenant le précurseur de colorant d'oxydation du type para ou du type ortho ou leur mélange et, dans une autre étape, à appliquer une composition tinctoriale contenant le coupleur de formule (I), l'agent oxydant étant introduit, juste avant l'application, dans la composition appliquée dans le deuxième temps ou bien être appliqué sur les fibres kératiniques, dans un troisième temps, les conditions de pose, de séchage et de lavage étant similaires à celles indiquées ci-dessus.

L'invention sera mieux comprise à l'aide des exemples ci-après.

Les dénominations commerciales "ETHOMEEN", "EUTANOL", "SIPON", "SIMULSOL" et "COMPERLAN" qui apparaissent dans ces exemples sont des marques déposées.

EXEMPLES D'APPLICATION

EXEMPLES 1 à 4

**COLORATION D'OXYDATION A pH ACIDE**

On prépare la composition suivante :

| | | | |
|---|---|---|---|
| - Alcool oléique polyglycérolé à 2 moles de glycérol | | 4,0 g | |
| - Alcool oléique polyglycérolé à 4 moles de glycérol à 78% de MA | | 5,69 g | MA |
| - Acide oléique | | 3,0 g | |
| - Amine oléique à 2 moles d'oxyde d'éthylène vendue sous la dénomination ETHOMEEN O 12 par la Société AKZO | | 7,0 g | |
| - Laurylaminosuccinamate de diéthylamino-propyle, sel de sodium à 55% de MA | | 3,0 g | MA |
| - Alcool oléique | | 5,0 g | |
| - Diéthanolamide d'acide oléique | | 12,0 g | |
| - Propylèneglycol | | 3,5 g | |
| - Alcool éthylique | | 7,0 g | |
| - Dipropylèneglycol | | 0,5 g | |
| - Monométhyléther de propylèneglycol | | 9,0 g | |
| - Métabisulfite de sodium en solution aqueuse à 35% de MA | | 0,45 g | MA |
| - Acétate d'ammonium | | 0,8 g | |
| - Antioxydant | qs | | |
| - Séquestrant | qs | | |
| - Parfum | qs | | |
| - Conservateurs | qs | | |
| - Monoéthanolamine | qsp pH=9,8 | | |
| - Colorants | | x g | |
| - Eau déminéralisée | qsp | 100,0 g | |

MA = matière active

La composition ci-dessus est mélangée, poids pour poids, avec de l'eau oxygénée à 20 volumes (6% en poids) dont le pH est ajusté entre 1 et 1,5 avec 2,5 g d'acide orthophosphorique.

Ce mélange a un pH d'environ 6,5.

Le mélange est appliqué, soit sur des cheveux gris naturels à 90% de blancs (N), soit sur des cheveux gris permanentés à 90% de blancs (P), à raison de 28 g de mélange pour 3 g de cheveux. On laisse poser pendant 30 minutes. Les cheveux sont ensuite rincés à l'eau et lavés avec un shampooing, puis séchés.

Le poids x des colorants (hydroxybenzofurannes de formule (I) et précurseur de colorants d'oxydation)) et les colorations obtenues, figurent dans le tableau (I) ci-dessous :

## TABLEAU I

| COLORANTS | EX. 1 | EX. 2 | EX. 3 | EX. 4 |
|---|---|---|---|---|
| 4-hydroxy benzofuranne | 0,402 g | | | |
| 5-hydroxy benzofuranne | | 0,402 g | | |
| 6-hydroxy benzofuranne | | | 0,402 g | |
| 7-hydroxy benzofuranne | | | | 0,402 g |
| 2,6-diméthyl p-phénylène diamine | 0,627 g | | | 0,627 g |
| p-phénylène diamine | | 0,324 g | 0,324 g | |
| Coloration obtenue sur cheveux naturels (N) | cendré bleu mat | | blond foncé cendré doré très mat | |
| Coloration obtenue sur cheveux permanentés (P) | | châtain clair cendré mat | | cendré mat |

EXEMPLE 5 à 7

**COLORATION D'OXYDATION A pH BASIOUE**

On prépare la composition suivante :

| | | |
|---|---|---|
| - Octyldodécanol vendu sous la dénomination EUTANOL G par la Société HENKEL | | 8,0 g |
| - Acide oléique | | 20,0 g |
| - Lauryléthersulfate de monoéthanolamine vendu sous la dénomination SIPON LM 35 par la Société HENKEL | | 3,0 g |
| - Alcool éthylique | | 10,0 g |
| - Alcool benzylique | | 10,0 g |
| - Alcool cétylstéarylique à 33 moles d'oxyde d'éthylène vendu sous la dénomination SIMULSOL GS par la Société SEPPIC | | 2,4 g |
| - Acide éthylènediamine tétracétique | | 0,2 g |
| - Solution aqueuse à 60% de MA d'un polymère cationique contenant des motifs de formule : 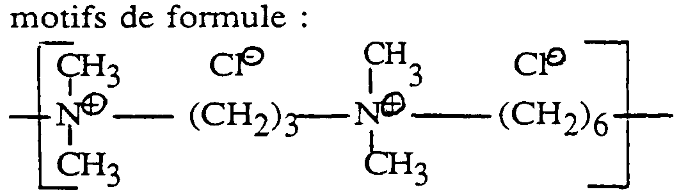 | | 3,7 g |
| - Monoéthanolamine | | 7,5 g |
| - Diéthanolamide d'acide linoléique vendu sous la dénomination COMPERLAN F par la Sociét HENKEL | | 8,0 g |
| - Ammoniaque à 20% de $NH_3$ | | 10,2 g |
| - Métabisulfite de sodium en solution aqueuse à 35% | | 1,3 g |
| - Hydroquinone | | 0,15 g |
| - 1-phényl 3-méthyl 5-pyrazolone | | 0,2 g |
| - Colorants | | x g |
| - Eau déminéralisée | qsp | 100,0 g |

La composition ci-dessus est mélangée, poids pour poids, avec de l'eau oxygénée à 20 volumes (6% en poids) dont le pH est égal à 3. Le mélange ainsi réalisé a un pH d'environ 9,5 et est appliqué, soit sur des cheveux gris naturels à 90% de blancs (N), soit sur des cheveux gris permanentés à 90% de blancs (P), à raison de 28 g de mélange pour 3 g de cheveux. On laisse poser pendant 30 minutes. Les cheveux

sont ensuite rincés à l'eau, lavés avec un shampooing puis séchés.

Les colorations obtenues sont regroupées dans le tableau (II) ci-après.

## TABLEAU II

| COLORANTS | EXEMPLE 5 | EXEMPLE 6 | EXEMPLE 7 |
|---|---|---|---|
| 4-hydroxy benzofuranne | 0,536 g | | |
| 6-hydroxy benzofuranne | | 0,268 g | |
| 7-hydroxy benzofuranne | | | 0,268 g |
| p-aminophénol | 0,436 g | | |
| p-phénylène diamine | | 0,216 g | 0,216 g |
| Coloration obtenue sur cheveux naturels (N) | blond clair très nacré | blond clair naturel cendré mat | |
| Coloration obtenue sur cheveux permanentés (P) | | | beige rosé |

EXEMPLE 8

**COLORATION D'OXYDATION ACIDE**

On prépare la composition suivante :

| | | | |
|---|---|---|---|
| - Alcool oléique polyglycérolé à 2 moles de glycérol | 4,0 | g | |
| - Alcool oléique polyglycérolé à 4 moles de glycérol à 78% de MA | 5,69 | g | MA |
| - Acide oléique | 3,0 | g | |
| - Amine oléique oxyéthylénée avec 2 moles d'oxyde d'éthylène, vendue sous la dénomination ETHOMEEN 0 12 par la Société AKZO | 7,0 | g | |
| - Laurylaminosuccinamate de diéthylamino-propyle, sel de sodium à 55% de MA | 3,0 | g | MA |
| - Alcool oléique | 5,0 | g | |
| - Diéthanolamide d'acide oléique | 12,0 | g | |
| - Propylèneglycol | 3,5 | g | |
| - Alcool éthylique | 7,0 | g | |
| - Dipropylèneglycol | 0,5 | g | |
| - Monométhyléther de propylèneglycol | 9,0 | g | |
| - Métabisulfite de sodium en solution aqueuse à 35% de MA | 0,45 | g | MA |
| - Acétate d'ammonium | 0,8 | g | |
| - Antioxydant qs | | | |
| - Séquestrant qs | | | |
| - Parfum qs | | | |
| - Conservateur qs | | | |
| - Monoéthanolamine qsp pH=9,8 | | | |
| - 3-méthyl 6-hydroxybenzofuranne | 0,45 | g | |
| - Dichlorhydrate de 2,6-diméthyl p-phénylènediamine | 0,63 | g | |
| - Eau déminéralisée qsp | 100,0 | g | |

La composition obtenue est mélangée, poids pour poids, avec de l'eau oxygénée à 20 volumes, dont le pH est ajusté entre 1 et 1,5 par de l'acide phosphorique (2,5 g d'acide phosphorique pour 100 g d'eau oxygénée).

Le mélange ainsi réalisé a un pH d'environ 6,5. Il est appliqué sur des cheveux gris permanentés pendant 30 minutes à température ambiante. Après rinçage et lavage, les cheveux sont teints en blond

doré mat.

EXEMPLES 9 et 10

**COLORATION D'OXYDATION ALCALINE**

On prépare les compositions suivantes :

| | | |
|---|---|---|
| - Octyldodécanol vendu sous la dénomination EUTANOL D par la Société HENKEL | 8,0 | g |
| - Acide oléique | 20,0 | g |
| - Lauryléthersulfate de monoéthanolamine vendu sous la dénomination SIPON LM 35 par la Société HENKEL | 3,0 | g |
| - Alcool éthylique | 10,0 | g |
| - Alcool benzylique | 10,0 | g |
| - Alcool cétylstéarylique à 33 moles d'oxyde d'éthylène vendu sous la dénomination SIMULSOL GS par la Société SEPPIC | 2,4 | g |
| - Acide éthylènediamine tétracétique | 0,2 | g |
| - Solution aqueuse à 60% de MA d'un polymère cationique contenant des motifs de formule : | 3,7 | g |

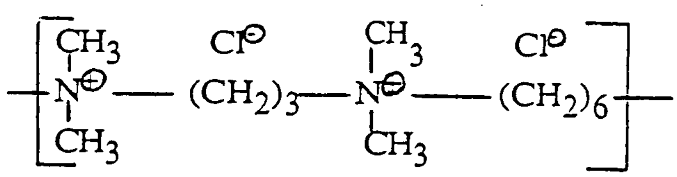

| | | | |
|---|---|---|---|
| - Monoéthanolamine | | 7,5 | g |
| - Diéthanolamide d'acide linoléique vendu sous la dénomination COMPERLAN F par la Société HENKEL | | 8,0 | g |
| - Ammoniaque à 20% de $NH_3$ | | 10,2 | g |
| - Métabisulfite de sodium en solution aqueuse à 35% | | 1,3 | g |
| - Hydroquinone | | 0,15 | g |
| - 1-phényl 3-méthyl 5-pyrazolone | | 0,2 | g |
| - Hydroxybenzofurannes et précurseurs de colorants d'oxydation indiqués dans le tableau | | | |
| - Eau déminéralisée | qsp | 100,0 | g |

La composition obtenue est mélangée poids pour poids avec de l'eau oxygénée à 20 volumes et dont le pH est égal à 3. Le mélange a un pH d'environ 9,5. Il est appliqué sur des cheveux gris permanentés pendant 30 minutes. Après rinçage et lavage, les cheveux sont teints dans la couleur indiquée dans le tableau.

| Exemples | Hydroxybenzofurannes et Précurseurs de colorants | | Couleur des cheveux teints |
|---|---|---|---|
| 9 | - 3-méthyl 6-hydroxybenzofuranne<br>- p-phénylènediamine | 0,30 g<br>0,22 g | Blond naturel cendré |
| 10 | - 3-méthyl 6-hyroxybenzofuranne<br>- 4-amino 2-méthoxyméthylphénol | 0,89 g<br>0,92 g | Blond clair doré mat |

**Revendications**

1. Composition tinctoriale pour la teinture des fibres kératiniques et en particulier des cheveux humains, caractérisée par le fait qu'elle comprend, dans un véhicule aqueux ou hydroalcoolique en tant que coupleur, un ou plusieurs hyroxybenzofurannes de formule (I) :

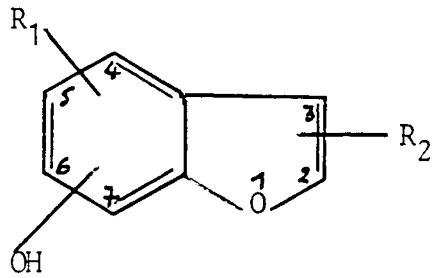

(I)

dans laquelle $R_1$ et $R_2$, identiques ou différents, représentent un atome d'hydrogène ou un radical alkyle en $C_1$-$C_6$. $R_1$ et $R_2$ peuvent occuper n'importe quelle position du noyau benzofuranne avec la condition que lorsque $R_1$ et $R_2$ désignent tous deux un radical alkyle, ils n'occupent pas à la fois les positions 2 et 3; en association avec un ou plusieurs précurseurs de colorants d'oxydation choisis parmi les précurseurs de colorants d'oxydation du type para et les précurseurs de colorants d'oxydation du type ortho et leurs mélanges.

**2.** Composition tinctoriale selon la revendication 1, caractérisée par le fait que l'hydroxybenzofuranne de formule (I) est choisi dans le groupe suivant :

- le 2-méthyl 6-hydroxybenzofuranne,
- le 3-méthyl 6-hydroxybenzofuranne,
- le 2,4-diméthyl 6-hydroxybenzofuranne,
- le 3-n-propyl 6-hydroxybenzofuranne,
- le 2-éthyl 5-hydroxybenzofuranne,
- le 2-méthyl 5-hydroxybenzofuranne,
- le 3-méthyl 5-hydroxybenzofuranne,
- le 3-isobutyl 5-hydroxybenzofuranne,
- le 3-éthyl 5-hydroxybenzofuranne,
- le 2,6-diméthyl 5-hydroxybenzofuranne,
- le 3,6-diméthyl 5-hydroxybenzofuranne,
- le 6,7-diméthyl 5-hydroxybenzofuranne,
- le 3-n-propyl 5-hydroxybenzofuranne,
- le 3-méthyl 4-n-propyl 5-hydroxybenzofuranne,
- le 2-hexyl 5-hydroxybenzofuranne,
- le 2-n-propyl 5-hydroxybenzofuranne,
- le 4-tertiobutyl 5-hydroxybenzofuranne,
- le 6-tertiobutyl 5-hydroxybenzofuranne,
- le 4-méthyl 5-hydroxybenzofuranne,
- le 3-isobutyl 5-hydroxybenzofuranne,
- le 3-méthyl 5-n-propyl 4-hydroxybenzofuranne,
- le 2-éthyl 4-hydroxybenzofuranne,
- le 2-méthyl 6-pentyl 4-hydroxybenzofuranne,
- le 6-pentyl 4-hydroxybenzofuranne,
- le 3,5-diméthyl 4-hydroxybenzofuranne,
- le 3,7-diméthyl 4-hydroxybenzofuranne,
- le 2,6-di-tertiobutyl 4-hydroxybenzofuranne,
- le 2-méthyl 4-hydroxybenzofuranne,
- le 3-méthyl 4-hydroxybenzofuranne,
- le 2-méthyl 7-éthyl 4-hydroxybenzofuranne,
- le 2,7-diméthyl 4-hydroxybenzofuranne,
- le 2-isopropyl 4-hydroxybenzofuranne,
- le 3-éthyl 4-hydroxybenzofuranne,
- le 3-méthyl 7-tertiobutyl 4-hydroxybenzofuranne,
- le 3-méthyl 5-tertiobutyl 4-hydroxybenzofuranne,
- le 2,6-diméthyl 4-hydroxybenzofuranne,
- le 3-isopropyl 4-hydroxybenzofuranne,
- le 3-n-propyl 4-hydroxybenzofuranne,

- le 3-méthyl 7-n-propyl 4-hydroxybenzofuranne,
- le 3-méthyl 6-n-propyl 7-hydroxybenzofuranne,
- le 3-méthyl 7-hydroxybenzofuranne,
- le 2-éthyl 4-méthyl 7-hydroxybenzofuranne,
- le 2-éthyl 5-méthyl 7-hydroxybenzofuranne.

**3.** Composition tinctoriale selon la revendication 2, caractérisée par le fait que l'hydroxybenzofuranne est choisi dans le groupe formé par le 3-méthyl 6-hydroxybenzofuranne et les 4-, 5-, 6- et 7-hydroxy benzofurannes.

**4.** Composition tinctoriale selon les revendications 1 à 3, caractérisée par le fait que le précurseur de colorant d'oxydation de type para est choisi parmi les paraphénylènediamines, les paraaminophénols, les composés hétérocycliques du type para, tels que la 2,5-diaminopyridine, la 2-hydroxy 5-aminopyridine, la tétraaminopyridine, les bases doubles et leurs mélanges.

**5.** Composition tinctoriale selon la revendication 4, caractérisée par le fait que les paraphénylènediamines répondent à la formule :

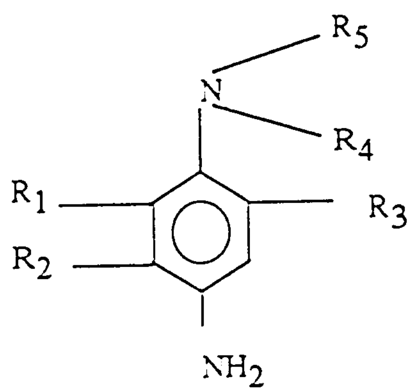

(II)

dans laquelle $R_1$, $R_2$ et $R_3$, identiques ou différents, représentent un atome d'hydrogène ou d'halogène, un radical alkyle ayant 1 à 4 atomes de carbone, un radical alcoxy ayant 1 à 4 atomes de carbone, $R_4$ et $R_5$, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, pipéridinoalkyle, morpholinoalkyle, phényle, phényle substitué en para par un groupe amino, ces groupes alkyle ou alcoxy ayant de 1 à 4 atomes de carbone, ou bien $R_4$ et $R_5$ forment, conjointement avec l'atome d'azote auquel ils sont liés, un hétérocycle pipéridino ou morpholino, sous réserve que $R_1$ ou $R_3$ représente un atome d'hydrogène lorsque $R_4$ et $R_5$ ne représentent pas un atome d'hydrogène, ainsi que les sels de ces composés.

**6.** Composition tinctoriale selon la revendication 4, caractérisée par le fait qu'elle contient au moins une paraphénylènediamine choisie dans le groupe formé par la p-phénylènediamine, la p-toluylène diamine, la méthoxyparaphénylènediamine, la chloroparaphénylène diamine, la 2,6-diméthylparaphénylènediamine, la 2,6-diéthyl paraphénylènediamine, la 2,5-diméthylparaphénylènediamine, la 2-méthyl 5-méthoxyparaphénylènediamine, la 2,6-diméthyl 5-méthoxyparaphénylènediamine, la N,N-diméthylparaphénylène diamine, la N,N-diéthylparaphénylènediamine, la N,N-dipropylparaphénylènediamine, la 3-méthyl 4-amino N,N-diéthylaniline, la N,N-di (β-hydroxyéthyl)paraphénylènediamine, la 3-méthyl 4-amino N,N-di-(β-hydroxyéthyl)aniline, la 3-chloro 4-amino N,N-di-(β-hydroxyéthyl) aniline, la 4-amino N,N-(éthyl, carbamylméthyl)aniline, la 3-méthyl 4-amino N,N-(éthyl, carbamylméthyl)aniline, la 4-amino N,N-(éthyl, β-pipéridinoéthyl)aniline, la 3-méthyl 4-amino N,N-(éthyl, β-pipéridinoéthyl)aniline, la 4-amino N,N-(éthyl, β-morpholinoéthyl)aniline, la 3-méthyl 4-amino N,N-(éthyl, β-morpholinoéthyl)aniline, la 4-amino N,N-(éthyl,β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-(éthyl,β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3-méthyl 4-amino N,N-(éthyl,β-sulfoéthyl)aniline, la N-[(4′-amino)phényl]morpholine, la N-[(4′-amino)phényl]pipéridine, la

2-hydroxyéthylparaphénylènediamine, la fluoroparaphénylènediamine, la carboxyparaphénylènediamine, la sulfoparaphénylènediamine, la 2-isopropylparaphénylènediamine, la 2-n-propylparaphénylènediamine, l'hydroxy-2-n-propylparaphénylène diamine, la 2-hydroxyméthylparaphénylènediamine, la N,N-diméthyl 3-méthylparaphénylènediamine, la N,N-(éthyl,$\beta$-hydroxyéthyl)paraphénylènediamine, la N-(dihydroxypropyl)paraphénylènediamine, la N-4′-aminophénylparaphénylène diamine, la N-phénylparaphénylène diamine, sous forme de base libre ou sous forme de sel cosmétiquement acceptable.

**7.** Composition tinctoriale selon la revendication 4, caractérisée par le fait qu'elle contient au moins un p-aminophénol choisi dans le groupe formé par le p-aminophénol, le 2-méthyl 4-aminophénol, le 3-méthyl 4-aminophénol, le 2-chloro 4-aminophénol, le 3-chloro 4-aminophénol, le 2,3-diméthyl 4-aminophénol, le 2-hydroxyméthyl 4-aminophénol, le 2-($\beta$-hydroxyéthyl)4-aminophénol, le 2-méthoxy 4-aminophénol, le 3-méthoxy 4-aminophénol, le 2,5-diméthyl 4-aminophénol, le 2-méthoxyméthyl 4-aminophénol, le 2-aminométhyl 4-aminophénol et le 2-$\beta$-hydroxyéthylaminométhyl 4-aminophénol.

**8.** Composition tinctoriale selon les revendications 1 à 3, caractérisée par le fait que le précurseur de colorant d'oxydation du type ortho est choisi dans le groupe formé par les orthoaminophénols, tels que le 1-amino 2-hydroxybenzène, le 6-méthyl 1-hydroxy 2-aminobenzène, le 4-méthyl 1-amino 2-hydroxybenzène, les orthophénylènediamines.

**9.** Composition tinctoriale selon la revendication 4, caractérisée par le fait que le précurseur de colorant d'oxydation du type para est choisi dans le groupe formé par des bases dites doubles qui sont des bisphénylènealkylènediamines, répondant à la formule :

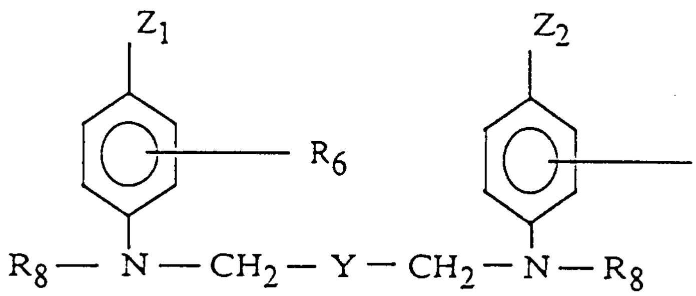

(III)

dans laquelle :

$Z_1$ et $Z_2$, identiques ou différents, représentent des groupements hydroxyle ou $NHR_9$, où $R_9$ désigne un atome d'hydrogène ou un radical alkyle inférieur;

$R_6$ et $R_7$, identiques où différents, représentent des atomes d'hydrogène, des atomes d'halogène ou encore des groupements alkyle;

$R_8$ représente un atome d'hydrogène, un groupe alkyle, hydroxy alkyle ou aminoalkyle, dont le reste amino peut être substitué par un ou deux groupements alkyle;

Y représente un radical choisi parmi les radicaux suivants:

$-(CH_2)_n-$, $(CH_2)_m-O-(CH_2)_m$,
$-(CH_2)_m-CHOH-(CH_2)_m$,

$$-(CH_2)_m-\underset{\displaystyle CH_3}{\underset{|}{N}}-(CH_2)_m;$$

n est un nombre entier compris entre 0 et 8 et m un nombre entier compris entre 0 et 4, ces bases dites doubles pouvant se présenter également sous forme de leurs sels d'addition avec des acides; les radicaux alkyle ou alcoxy ci-dessus indiqués désignent un groupement ayant 1 à 4 atomes de carbone.

**10.** Composition tinctoriale selon la revendication 9, caractérisée par le fait que les bases dites doubles sont choisies parmi le N,N′-bis-($\beta$-hydroxyéthyl)N,N′-bis-(4′-aminophényl)1,3-diamino 2-propanol, la

N,N′-bis-(β-hydroxyéthyl)N,N′-bis-(4′-aminophényl)éthylènediamine, la N,N′-bis-(4-aminophényl)-tétraméthylènediamine, la N,N′-bis-(β-hydroxyéthyl)N,N′-bis-(4-aminophényl)tétraméthylènediamine, la N,N′-bis-(4-méthylaminophényl)tétraméthylènediamine, la N,N′-bis-(ethyl)N,N′-bis-(4′-amino3′-méthyl phényl)éthylènediamine.

**11.** Composition tinctoriale selon les revendications 1 à 10, caractérisée par le fait qu'elle contient également d'autres coupleurs choisis dans le groupe formé par les métadiphénols, les métaaminophénols, les métaphénylènediamines, les métaacylaminophénols, les métauréidophénols, les métacarbalcoxyaminophénols, l'α-naphtol, les coupleurs possédant un groupement méthylène actif, tels que les composés β-cétoniques, les pyrazolones, les coupleurs hétérocycliques et le 4-, 6-, ou 7-hydroxyindole.

**12.** Composition tinctoriale selon la revendication 11, caractérisée par le fait qu'elle contient des coupleurs choisis dans le groupe formé par le 2,4-dihydroxyphénoxyéthanol, le 2,4-dihydroxyanisole, le métaaminophénol, le monométhyléther de résorcine, le 2-méthyl 5-N-(β-hydroxyéthyl)aminophénol, le 2-méthyl 5-N-(β-mésylamino éthyl)aminophénol, la 6-hydroxybenzomorpholine, le 2,4-diaminoanisole, le 2,4-diaminophénoxyéthanol, la 6-aminobenzomorpholine, le [2-N-(β-hydroxyéthyl) amino 4-amino]-phénoxyéthanol, le 2-amino 4-N-(β-hydroxyéthyl) amino anisole, le (2,4-diamino)phényl-β-γ-dihydroxypropyléther, la 2,4-diamino phénoxyéthylamine, le 1,3-diméthoxy 2,4-diaminobenzène, le 2-méthyl 5-aminophénol, le 2,6-diméthyl 3-aminophénol, le 1-amino 3,4-méthylènedioxybenzène, le 1-hydroxy 3,4-méthylènedioxybenzène, le 2-chloro 6-méthyl 3-aminophénol, le 2-méthyl 3-aminophénol, le 2-chlororésorcinol, le résorcinol, la 6-méthoxy 3-hydroxyéthylamino aniline, le 1-éthoxy 2-bis-(β-hydroxyéthyl)-amino 4-aminobenzène, le 3-diéthylaminophénol, le 1,3-dihydroxy 2-méthylbenzène, le 1-hydroxy 2,4-dichloro 3-aminobenzène, le 4,6-hydroxyéthoxy 1,3-diaminobenzène, le 4-méthyl 6-éthoxy 1,3-diaminobenzène, le 4-chloro 6-méthyl 3-aminophénol, le 6-chloro 3-trifluoroéthylamino phénol, leurs sels et leurs mélanges.

**13.** Composition tinctoriale selon les revendications 1 à 12, caractérisée par le fait que la concentration en composés de formule (I) est de 0,05 à 3,5% en poids du poids total de la composition.

**14.** Composition tinctoriale selon les revendications 1 à 13, caractérisée par le fait que la concentration totale en coupleurs et en précurseurs de colorants d'oxydation de type para et du type ortho est de 0,3 à 7% en poids du poids total de la composition.

**15.** Composition tinctoriale selon les revendications 1 à 14, caractérisée par le fait qu'elle contient en outre des colorants directs choisis parmi les colorants azoïques, anthraquinoniques et les dérivés nitrés de la série benzénique.

**16.** Composition tinctoriale selon les revendications 1 à 15, caractérisée par le fait qu'elle a un pH compris entre 4 et 11.

**17.** Composition tinctoriale selon les revendications 1 à 16, caractérisée par le fait qu'elle contient de 1 à 40% en poids d'un solvant organique choisi parmi les alcanols inférieurs en $C_1$-$C_4$, le glycérol, les glycols, les éthers de glycol, les alcools aromatiques tels que l'alcool benzylique ou le phénoxyéthanol et leurs mélanges.

**18.** Composition tinctoriale selon les revendications 1 à 17, caractérisée par le fait qu'elle contient également de 0,5 à 55% en poids d'un ou plusieurs agents tensio- actifs anioniques, cationiques, non-ioniques, amphotères ou leurs mélanges.

**19.** Composition tinctoriale selon les revendications 1 à 18, caractérisée par le fait qu'elle contient en outre des adjuvants cosmétiques choisis parmi les épaississants, les antioxydants, les agents de pénétration, les agents séquestrants, les tampons, les parfums, les agents alcalinisants et les propulseurs.

**20.** Composition tinctoriale selon les revendications 1 à 19, caractérisée par le fait qu'elle se présente sous forme de liquides, de crèmes, de gels ou est conditionnée sous forme d'aérosol en présence d'un agent propulseur.

**21.** Procédé de teinture des cheveux, par oxydation, caractérisé par le fait qu'il consiste à mélanger au moment de l'emploi une composition tinctoriale selon l'une quelconque des revendications 1 à 20, avec une solution oxydante en une quantité suffisante pour provoquer l'oxydation du ou des précurseurs de colorants d'oxydation, le pH du mélange étant compris entre 3,5 et 10, puis à appliquer le mélange obtenu sur les cheveux, à le laisser poser pendant 10 à 40 minutes, puis à rincer les cheveux, à les laver au shampooing, à les rincer à nouveau et à les sécher.

**22.** Procédé de teinture des cheveux par oxydation, à plusieurs étapes, caractérisé par le fait qu'il consiste, dans l'une des étapes, à appliquer sur les cheveux une composition tinctoriale contenant un ou plusieurs précurseurs de colorants d'oxydation de type para, de type ortho ou leurs mélanges, puis dans une autre étape, à appliquer une composition tinctoriale contenant l'hydroxybenzofuranne de formule (I) ou l'un de ses sels, l'agent oxydant étant présent dans la composition appliquée dans le deuxième temps ou bien l'agent oxydant étant appliqué sur les cheveux eux-mêmes, dans un troisième temps, à laisser poser pendant 10 à 40 minutes, puis à rincer les cheveux, à les laver au shampooing, à les rincer à nouveau et à les sécher.

**Claims**

**1.** Dyeing composition for dyeing keratinous fibres and in particular human hair, characterized in that it comprises, as coupler in an aqueous or dilute alcoholic vehicle, one or more hydroxybenzofurans of formula (I):

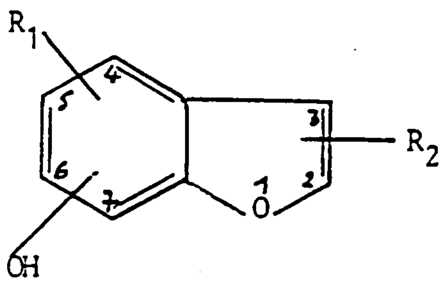

(I)

in which $R_1$ and $R_2$, which are identical or different, represent a hydrogen atom or a $C_1$-$C_6$ alkyl radical. $R_1$ and $R_2$ can occupy any position on the benzofuran ring provided that when $R_1$ and $R_2$ both denote an alkyl radical, they do not occupy the positions 2 and 3 at the same time; in combination with one or more oxidation dye precursors chosen from the *para* type oxidation dye precursors and the *ortho* type oxidation dye precursors and mixtures thereof.

**2.** Dyeing composition according to Claim 1, characterized in that the hydroxybenzofuran of formula (I) is chosen from the following group:

- 2-methyl-6-hydroxybenzofuran,
- 3-methyl-6-hydroxybenzofuran,
- 2,4-dimethyl-6-hydroxybenzofuran,
- 3-n-propyl-6-hydroxybenzofuran,
- 2-ethyl-5-hydroxybenzofuran,
- 2-methyl-5-hydroxybenzofuran,
- 3-methyl-5-hydroxybenzofuran,
- 3-isobutyl-5-hydroxybenzofuran,
- 3-ethyl-5-hydroxybenzofuran,
- 2,6-dimethyl-5-hydroxybenzofuran,
- 3,6-dimethyl-5-hydroxybenzofuran,
- 6,7-dimethyl-5-hydroxybenzofuran,
- 3-n-propyl-5-hydroxybenzofuran,
- 3-methyl-4-n-propyl-5-hydroxybenzofuran,
- 2-hexyl-5-hydroxybenzofuran,
- 2-n-propyl-5-hydroxybenzofuran,
- 4-tert-butyl-5-hydroxybenzofuran,

- 6-tert-butyl-5-hydroxybenzofuran,
- 4-methyl-5-hydroxybenzofuran,
- 3-isobutyl-5-hydroxybenzofuran,
- 3-methyl-5-n-propyl-4-hydroxybenzofuran,
- 2-ethyl-4-hydroxybenzofuran,
- 2-methyl-6-pentyl-4-hydroxybenzofuran,
- 6-pentyl-4-hydroxybenzofuran,
- 3,5-dimethyl-4-hydroxybenzofuran,
- 3,7-dimethyl-4-hydroxybenzofuran,
- 2,6-di-tert-butyl-4-hydroxybenzofuran,
- 2-methyl-4-hydroxybenzofuran,
- 3-methyl-4-hydroxybenzofuran,
- 2-methyl-7-ethyl-4-hydroxybenzofuran,
- 2,7-dimethyl-4-hydroxybenzofuran,
- 2-isopropyl-4-hydroxybenzofuran,
- 3-ethyl-4-hydroxybenzofuran,
- 3-methyl-7-tert-butyl-4-hydroxybenzofuran,
- 3-methyl-5-tert-butyl-4-hydroxybenzofuran,
- 2,6-dimethyl-4-hydroxybenzofuran,
- 3-isopropyl-4-hydroxybenzofuran,
- 3-n-propyl-4-hydroxybenzofuran,
- 3-methyl-7-n-propyl-4-hydroxybenzofuran,
- 3-methyl-6-n-propyl-7-hydroxybenzofuran,
- 3-methyl-7-hydroxybenzofuran,
- 2-ethyl-4-methyl-7-hydroxybenzofuran,
- 2-ethyl-5-methyl-7-hydroxybenzofuran.

**3.** Dyeing composition according to Claim 2, characterized in that the hydroxybenzofuran is chosen from the group consisting of 3-methyl-6-hydroxybenzofuran and 4-, 5-, 6- and 7-hydroxybenzofurans.

**4.** Dyeing composition according to Claims 1 to 3, characterized in that the *para* type oxidation dye precursor is chosen from *para*-phenylenediamines, *para*-aminophenols, *para* type heterocyclic compounds such as 2,5-diaminopyridine, 2-hydroxy-5-aminopyridine, tetraaminopyrimidine, double bases and mixtures thereof.

**5.** Dyeing composition according to Claim 4, characterized in that the *para*-phenylenediamines are of the formula:

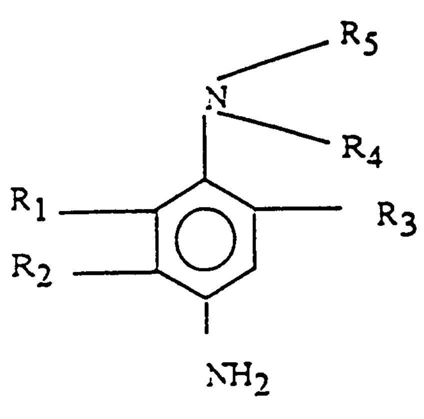

(II)

in which $R_1$, $R_2$ and $R_3$, which are identical or different, represent a hydrogen or halogen atom, an alkyl radical having 1 to 4 carbon atoms, an alkoxy radical having 1 to 4 carbon atoms, $R_4$ and $R_5$, which are identical or different, represent a hydrogen atom, an alkyl, hydroxyalkyl, alkoxyalkyl, carbamylalkyl, mesylaminoalkyl, acetylaminoalkyl, ureidoalkyl, carbalkoxyaminoalkyl, piperidinoalkyl, morpholinoalkyl or phenyl radical, or a phenyl radical substituted in the *para* position by an amino group, these alkyl or

alkoxy groups having 1 to 4 carbon atoms, or alternatively $R_4$ and $R_5$ form, together with the nitrogen atom to which they are attached, a piperidino or morpholino heterocycle, provided that $R_1$ or $R_3$ represents a hydrogen atom when $R_4$ and $R_5$ do not represent a hydrogen atom, as well as the salts of these compounds.

**6.** Dyeing composition according to Claim 4, characterized in that it contains at least one *para*-phenylenediamine chosen from the group consisting of *p*-phenylenediamine, *p*-tolylenediamine, methoxy-*para*-phenylenediamine, chloro-*para*-phenylenediamine, 2,6-dimethyl-*para*-phenylenediamine, 2,6-diethyl-*para*-phenylenediamine, 2,5-dimethyl-*para*-phenylenediamine, 2-methyl-5-methoxy-*para*-phenylenediamine, 2,6-dimethyl-5-methoxy-*para*-phenylenediamine, *N,N*-dimethyl-*para*-phenylenediamine, *N,N*-diethyl-*para*-phenylenediamine, *N,N*-dipropyl-*para*-phenylenediamine, 3-methyl-4-amino-*N,N*-diethylaniline, *N,N*-di(β-hydroxyethyl)-*para*-phenylenediamine, 3-methyl-4-amino-*N,N*-di(β-hydroxyethyl)aniline, 3-chloro-4-amino-*N,N*-di(β-hydroxyethyl)aniline, 4-amino-*N*-ethyl-*N*-carbamylmethylaniline, 3-methyl-4-amino-*N*-ethyl-*N*-carbamylmethylaniline, 4-amino-*N*-ethyl-*N*-β-piperidinoethylaniline, 3-methyl-4-amino-*N*-ethyl-*N*-β-piperidinoethylaniline, 4-amino-*N*-ethyl-*N*-β-morpholino-ethylaniline, 3-methyl-4-amino-*N*-ethyl-*N*-β-morpholinoethylaniline, 4-amino-*N*-ethyl-*N*-β-acetylaminoethylaniline, 4-amino-*N*-(β-methoxyethyl)aniline, 3-methyl-4-amino-*N*-ethyl-*N*-β-acetylaminoethylaniline, 4-amino-*N*-ethyl-*N*-β-mesylaminoethylaniline, 3-methyl-4-amino-*N*-ethyl-*N*-β-mesylaminoethylaniline, 4-amino-*N*-ethyl-*N*-β-sulphoethylaniline, 3-methyl-4-amino-*N*-ethyl-*N*-β-sulphoethylaniline, *N*-[(4'-amino)phenyl]morpholine, *N*-[(4'-amino)phenyl]piperidine, 2-hydroxyethyl-*para*-phenylenediamine, fluoro-*para*-phenylenediamine, carboxy-*para*-phenylenediamine, sulpho-*para*-phenylenediamine, 2-isopropyl-*para*-phenylenediamine, 2-*n*-propyl-*para*-phenylenediamine, hydroxy-2-*n*-propyl-*para*-phenylenediamine, 2-hydroxymethyl-*para*-phenylenediamine, *N,N*-dimethyl-3-methyl-*para*-phenylenediamine, *N*-ethyl-*N*-β-hydroxyethyl-*para*-phenylenediamine, *N*-(dihydroxypropyl)-*para*-phenylenediamine, *N*-4'-aminophenyl-*para*-phenylenediamine, *N*-phenyl-*para*-phenylenediamine, in the form of a cosmetically acceptable free base or salt.

**7.** Dyeing composition according to Claim 4, characterized in that it contains at least one *p*-aminophenol chosen from the group consisting of *p*-aminophenol, 2-methyl-4-aminophenol, 3-methyl-4-aminophenol, 2-chloro-4-aminophenol, 3-chloro-4-aminophenol, 2,3-dimethyl-4-aminophenol, 2-hydroxymethyl-4-aminophenol, 2-(β-hydroxyethyl)-4-aminophenol, 2-methoxy-4-aminophenol, 3-methoxy-4-aminophenol, 2,5-dimethyl-4-aminophenol, 2-methoxymethyl-4-aminophenol, 2-aminomethyl-4-aminophenol and 2-β-hydroxyethylaminomethyl-4-aminophenol.

**8.** Dyeing composition according to Claims 1 to 3, characterized in that the *ortho* type oxidation dye precursor is chosen from the group consisting of *ortho*-aminophenols such as 1-amino-2-hydroxybenzene, 6-methyl-1-hydroxy-2-aminobenzene, 4-methyl-1-amino-2-hydroxybenzene and *ortho*-phenylenediamines.

**9.** Dyeing composition according to Claim 4, characterized in that the *para* type oxidation dye precursor is chosen from the group consisting of the so-called double bases which are bis-phenylenealkylenediamines of the formula:

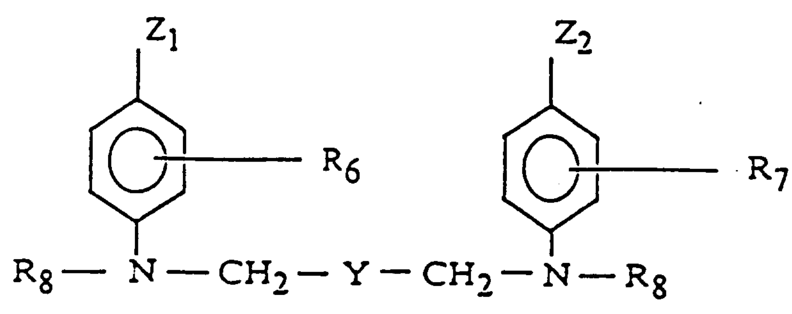

(III)

in which:

$Z_1$ and $Z_2$, which are identical or different, represent hydroxyl or $NHR_9$ groups, where $R_9$ denotes a hydrogen atom or a lower alkyl radical;

$R_6$ and $R_7$, which are identical or different, represent hydrogen atoms, halogen atoms or alternatively alkyl groups;

$R_8$ represents a hydrogen atom, an alkyl or hydroxyalkyl group, or an aminoalkyl group whose amino residue may be substituted by one or two alkyl groups;

Y represents a radical chosen from the following radicals:

$-(CH_2)_n-$, $(CH_2)_m-O-(CH_2)_m$,
$-(CH_2)_m-CHOH-(CH_2)_m$,

$$-(CH_2)_m-\underset{CH_3}{N}-(CH_2)_m;$$

n is an integer between 0 and 8 and m is an integer between 0 and 4, these so-called double bases being able also to exist in the form of their addition salts with acids, and the alkyl or alkoxy radicals indicated above denoting a group having 1 to 4 carbon atoms.

**10.** Dyeing composition according to Claim 9, characterized in that the so-called double bases are chosen from *N,N'*-bis(β-hydroxyethyl)-*N,N'*-bis(4'-aminophenyl)-1,3-diamino-2-propanol, *N,N'*-bis(β-hydroxyethyl)-*N,N'*-bis(4'-aminophenyl)ethylenediamine, *N,N'*-bis(4-aminophenyl)tetramethylenediamine, *N,N'*-bis(β-hydroxyethyl)-*N,N'*-bis(4-aminophenyl)tetramethylenediamine, *N,N'*-bis(4-methylaminophenyl)-tetramethylenediamine, *N,N'*-bis(ethyl)-*N,N'*-bis(4'-amino-3'-methylphenyl)ethylenediamine.

**11.** Dyeing composition according to Claims 1 to 10, characterized in that it also contains other couplers chosen from the group consisting of *meta*-diphenols, *meta*-aminophenols, *meta*-phenylenediamines, *meta*-acylaminophenols, *meta*-ureidophenols, *meta*-carbalkoxyaminophenols, α-naphthol, couplers containing an active methylene group such as β-ketonic compounds, pyrazolones, heterocyclic couplers and 4-, 6- or 7-hydroxyindole.

**12.** Dyeing composition according to Claim 11, characterized in that it contains couplers chosen from the group consisting of 2,4-dihydroxyphenoxyethanol, 2,4-dihydroxyanisole, *meta*-aminophenol, resorcinol monomethyl ether, 2-methyl-5-*N*-(β-hydroxyethyl)aminophenol, 2-methyl-5-*N*-(β-mesylaminoethyl)-aminophenol, 6-hydroxybenzomorpholine, 2,4-diaminoanisole, 2,4-diaminophenoxyethanol, 6-aminobenzomorpholine, [2-*N*-(β-hydroxyethyl)amino-4-amino]phenoxyethanol,2-amino-4-*N*-(β-hydroxyethyl)-aminoanisole, (2,4-diamino)phenyl-β,γ-dihydroxypropyl ether, 2,4-diaminophenoxyethylamine, 1,3-dimethoxy-2,4-diaminobenzene, 2-methyl-5-aminophenol, 2,6-dimethyl-3-aminophenol, 1-amino-3,4-methylenedioxybenzene, 1-hydroxy-3,4-methylenedioxybenzene, 2-chloro-6-methyl-3-aminophenol, 2-methyl-3-aminophenol, 2-chlororesorcinol, resorcinol, 6-methoxy-3-hydroxyethylaminoaniline, 1-ethoxy-2-bis(β-hydroxyethyl)amino-4-aminobenzene, 3-diethyl-aminophenol, 1,3-dihydroxy-2-methylbenzene, 1-hydroxy-2,4-dichloro-3-aminobenzene,4,6-hydroxyethoxy-1,3-diaminobenzene, 4-methyl-6-ethoxy-1,3-diaminobenzene, 4-chloro-6-methyl-3-aminophenol, 6-chloro-4-trifluoroethylaminophenol, the salts and mixtures thereof.

**13.** Dyeing composition according to Claims 1 to 12, characterized in that the concentration of compounds of formula (I) is 0.05 to 3.5% by weight of the total weight of the composition.

**14.** Dyeing composition according to Claims 1 to 13, characterized in that the total concentration of couplers and of *para* type and *ortho* type oxidation dye precursors is 0.3 to 7% by weight of the total weight of the composition.

**15.** Dyeing composition according to Claims 1 to 14, characterized in that it contains, in addition, direct dyes chosen from azo and anthraquinone dyes, and nitro derivatives of the benzene series.

**16.** Dyeing composition according to Claims 1 to 15, characterized in that it has a pH of between 4 and 11.

**17.** Dyeing composition according to Claims 1 to 16, characterized in that it contains 1 to 40% by weight of an organic solvent chosen from lower $C_1$-$C_4$ alkanols, glycerol, glycols, glycol ethers, aromatic alcohols

such as benzyl alcohol or phenoxyethanol and mixtures thereof.

**18.** Dyeing composition according to Claims 1 to 17, characterized in that it also contains 0.5 to 55% by weight of one or more anionic, cationic, nonionic or amphoteric surface-active agents or mixtures thereof.

**19.** Dyeing composition according to Claims 1 to 18, characterized in that it contains, in addition, cosmetic adjuvants chosen from thickeners, antioxidants, penetrating agents, sequestering agents, buffers, perfumes, alkalinizing agents and propellants.

**20.** Dyeing composition according to Claims 1 to 19, characterized in that it is provided in the form of liquids, creams, gels or packaged as an aerosol in the presence of a propelling agent.

**21.** Process for dyeing hair, by oxidation, characterized in that it consists in mixing at the time of use a dyeing composition according to any one of Claims 1 to 20, with an oxidizing solution in an amount which is sufficient to bring about the oxidation of the oxidation dye precursor(s), the pH of the mixture being between 3.5 and 10, and then applying the mixture obtained to hair, allowing it to act for 10 to 40 minutes, and then rinsing the hair, washing it with shampoo, rinsing it again and drying it.

**22.** Process for dyeing hair, by oxidation, in several stages, characterized in that it consists, in one of the stages, in applying to the hair a dyeing composition containing one or more *para* type or ortho type oxidation dye precursors or mixtures thereof, and then, in another stage, applying a dyeing composition containing the hydroxybenzofuran of formula (I) or one of its salts, the oxidizing agent being present in the composition applied in the second stage, or the oxidizing agent being applied to the hair itself in a third stage, allowing it to act for 10 to 40 minutes, and then rinsing the hair, washing it with shampoo, rinsing it again and drying it.

**Patentansprüche**

**1.** Färbezusammensetzung zur Färbung keratinischer Fasern und insbesondere menschlicher Haare, dadurch **gekennzeichnet**, daß
sie, in einem wässrigen oder hydroalkoholischen Trägermittel, als Kuppler ein oder mehrere Hydroxybenzofurane der Formel (I):

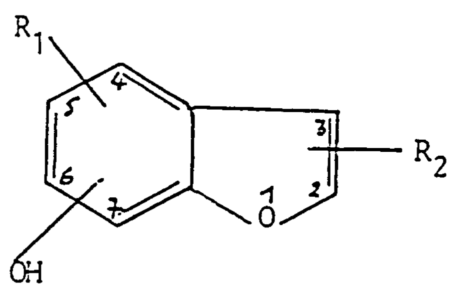

(I)

worin $R_1$ und $R_2$, gleich oder verschieden, ein Wasserstoffatom oder einen $C_{1-6}$-Alkylrest darstellen, wobei $R_1$ und $R_2$ irgendeine Position des Benzofurankerns besetzen können, mit der Maßgabe, daß, wenn alle beiden Reste $R_1$ und $R_2$ einen Alkylrest bedeuten, sie nicht gleichzeitig die Positionen 2 und 3 besetzen,
zusammen mit einer oder mehreren Vorstufen von Oxidationsfarbstoffen, ausgewählt aus Vorstufen von Oxidationsfarbstoffen des para-Typs und aus Vorstufen von Oxidationsfarbstoffen des ortho-Typs sowie aus deren Mischungen,
enthält.

**2.** Färbezusammensetzung gemäß Anspruch 1,
dadurch **gekennzeichnet**, daß das Hydroxybenzofuran der Formel (I) aus der folgenden Gruppe ausgewählt ist:

- 2-Methyl-6-hydroxybenzofuran,
- 3-Methyl-6-hydroxybenzofuran,
- 2,4-Dimethyl-6-hydroxybenzofuran,
- 3-n-Propyl-6-hydroxybenzofuran,
- 2-Ethyl-5-hydroxybenzofuran,
- 2-Methyl-5-hydroxybenzofuran,
- 3-Methyl-5-hydroxybenzofuran,
- 3-Isobutyl-5-hydroxybenzofuran,
- 3-Ethyl-5-hydroxybenzofuran,
- 2,6-Dimethyl-5-hydroxybenzofuran,
- 3,6-Dimethyl-5-hydroxybenzofuran,
- 6,7-Dimethyl-5-hydroxybenzofuran,
- 3-n-Propyl-5-hydroxybenzofuran,
- 3-Methyl-4-n-propyl-5-hydroxybenzofuran,
- 2-Hexyl-5-hydroxybenzofuran,
- 2-n-Propyl-5-hydroxybenzofuran,
- 4-t-Butyl-5-hydroxybenzofuran,
- 6-t-Butyl-5-hydroxybenzofuran,
- 4-Methyl-5-hydroxybenzofuran,
- 3-Isobutyl-5-hydroxybenzofuran,
- 3-Methyl-5-n-propyl-4-hydroxybenzofuran,
- 2-Ethyl-4-hydroxybenzofuran,
- 2-Methyl-6-pentyl-4-hydroxybenzofuran,
- 6-Pentyl-4-hydroxybenzofuran,
- 3,5-Dimethyl-4-hydroxybenzofuran,
- 3,7-Dimethyl-4-hydroxybenzofuran,
- 2,6-Di-t-butyl-4-hydroxybenzofuran,
- 2-Methyl-4-hydroxybenzofuran,
- 3-Methyl-4-hydroxybenzofuran,
- 2-Methyl-7-ethyl-4-hydroxybenzofuran,
- 2,7-Dimethyl-4-hydroxybenzofuran,
- 2-Isoprpyl-4-hydroxybenzofuran,
- 3-Ethyl-4-hydroxybenzofuran,
- 3-Methyl-7-t-butyl-4-hydroxybenzofuran,
- 3-Methyl-5-t-butyl-4-hydroxybenzofuran,
- 2,6-Dimethyl-4-hydroxybenzofuran,
- 3-Isopropyl-4-hydroxybenzofuran,
- 3-n-Propyl-4-hydroxybenzofuran,
- 3-Methyl-7-n-propyl-4-hydroxybenzofuran,
- 3-Methyl-6-n-propyl-7-hyroxybenzofuran,
- 3-Methyl-7-hydroxybenzofuran,
- 2-Ethyl-4-methyl-7-hydroxybenzofuran,
- 2-Ethyl-5-methyl-7-hydroxybenzofuran.

**3.** Färbezusammensetzung gemäß Anspruch 2,
dadurch **gekennzeichnet**, daß
das Hydroxybenzofuran aus der Gruppe aus 3-Methyl-6-hydroxybenzofuran und 4-, 5-, 6- und 7-Hydroxybenzofuranen ausgewählt ist.

**4.** Färbezusammensetzung gemaß einem der Ansprüche 1 bis 3,
dadurch **gekennzeichnet**, daß
die Oxidationsfarbstoff-Vorstufe vom para-Typ aus p-Phenylendiaminen, p-Aminophenolen, heterozyklischen Verbindungen des para-Typs, wie 2,5-Diaminopyridin, 2-Hydroxy-5-aminopyridin, Tetraaminopyrimidin, aus Doppelbasen sowie aus deren Mischungen ausgewählt ist.

**5.** Färbezusammensetzung gemäß Anspruch 4,
dadurch **gekennzeichnet**, daß
die p-Phenylendiamine die Formel aufweisen:

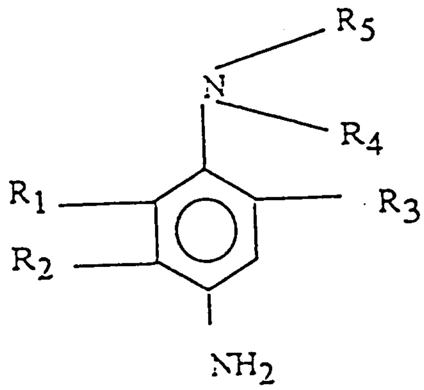

(II)

worin $R_1$, $R_2$ und $R_3$, gleich oder verschieden, ein Wasserstoff- oder Halogenatom, einen Alkylrest mit 1 bis 4 Kohlenstoffatomen oder einen Alkoxyrest mit 1 bis 4 Kohlenstoffatomen, $R_4$ und $R_5$, gleich oder verschieden, ein Wasserstoffatom, einen Alkyl-, Hydroxyalkyl-, Alkoxyalkyl-, Carbamylalkyl-, Mesylaminoalkyl-, Acetylaminoalkyl-, Ureidoalkyl-, Carbalkoxyaminoalkyl-, Piperidinoalkyl-, Morpholinoalkyl- oder Phenylrest darstellen, der gegenbenenfalls in para-Stellung mit einer Aminogruppe substituiert ist, wobei die jeweiligen Alkyl- oder Alkoxygruppen 1 bis 4 Kohlenstoffatome aufweisen, oder worin $R_4$ und $R_5$ zusammen mit dem Stickstoffatom, an das sie gebunden sind, eine Piperidino- oder Morpholino-Heterozyklus bilden, mit der Maßgabe, daß $R_1$ oder $R_2$ ein Wasserstoffatom darstellen, wenn $R_4$ und $R_5$ kein Wasserstoffatom darstellen, sowie durch die Salze dieser Verbindungen dargestellt sind.

**6.** Färbezusammensetzung gemäß Anspruch 4,
dadurch **gekennzeichnet**, daß
sie mindestens ein p-Phenylendiamin, ausgewählt aus der Gruppe aus p-Phenylendiamin, p-Toluylendiamin, Methoxy-p-phenylendiamin, Chlor-p-phenylendiamin, 2,6-Dimethyl-p-phenylendiamin, 2,6-Diethyl-p-phenylendiamin, 2,5-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, N,N-Diethyl-p-phenylendiamin, N,N-Dipropyl-p-phenylendiamin, 3-Methyl-4-amino-N,N-diethylanilin, N,N-Di(β-hydroxyethyl)-p-phenylendiamin, 3-Methyl-4-amino-N,N-di(β-hydroxyethyl)anilin, 3-Chlor-4-amino-N,N-di(β-hydroxyethyl)anilin, 4-Amino-N,N-(ethyl,carbamylmethyl)anilin, 3-Methyl-4-amino-N,N-(ethyl,carbamylmethyl)anilin, 4-Amino-N,N-(ethyl,β-piperidinoethyl)anilin, 3-Methyl-4-amino-N,N-(ethyl,β-piperdinoethyl)anilin, 4-Amino-N,N-(ethyl,β-morpholinoethyl)anilin, 3-Methyl-4-amino-N,N-(ethyl,β-morpholinoethyl)anilin, 4-Amino-N,N-(ethyl,β-acetylaminoethyl)anilin, 4-Amino-N-(β-methoxyethyl)anilin, 3-Methyl-4-amino-N,N-(ethyl,β-acetylaminoethyl)anilin, 4-Amino-N,N-(ethyl,β-mesylaminoethyl)anilin, 3-Methyl-4-amino-N,N-(ethyl,β-mesylaminoethyl)anilin, 4-Amino-N,N-(ethyl,β-sulfoethyl)anilin, 3-Methyl-4-amino-N,N-(ethyl,β-sulfoethyl)anilin, N-((4'-Amino)phenyl)morpholin, N-((4'-Amino)phenyl)piperidin, 2-Hydroxyethyl-p-phenylendiamin, Fluor-p-phenylendiamin, Carboxy-p-phenylendiamin, Sulfo-p-phenylendiamin,2-Isopropyl-p-phenylendiamin, 2-n-Propyl-p-phenylendiamin, Hydroxy-2-n-propyl-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(Dihydroxypropyl)-p-phenylendiamin, N-4'-Aminophenyl-p-phenylendiamin, N-Phenyl-p-phenylendiamin , in Form der freien Base oder in Form eines kosmetisch geeigneten Salzes enthält.

**7.** Färbezusammensetzung gemäß Anspruch 4,
dadurch **gekennzeichnet**, daß
sie mindestens ein p-Aminophenol enthält, ausgewählt aus der Gruppe aus p-Aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2-Hydroxymethyl-4-aminophenol, 2-(β-Hydroxyethyl)-4-aminophenol, 2-Methoxy-4-aminophenol, 3-Methoxy-4-aminophenol, 2,5-Dimethyl-4-aminophenol, 2-Methoxymethyl-4-aminophenol, 2-Aminomethyl-4-aminophenol und 2-β-Hydroxyethylaminomethyl-4-aminophenol.

**8.** Färbezusammensetzung gemäß einem der Ansprüche 1 bis 3,
dadurch **gekennzeichnet**, daß
die Oxidationsfarbstoff-Vorstufe des ortho-Typs aus der Gruppe aus o-Aminophenolen, wie 1-Amino-2-hydroxybenzol, 6-Methyl-1-hydroxy-2-aminobenzol, 4-Methyl-1-amino-2-hydroxybenzol, und aus o-Phenylendiaminen ausgewählt ist.

**9.** Färbezusammensetzung gemäß Anspruch 4, dadurch **gekennzeichnet**, daß die Oxidationsfarbstoff-Vorstufe des para-Typs aus der Gruppe aus doppelte genannten Basen ausgewählt ist, welche Bisphenylendialkylendiamine der Formel sind:

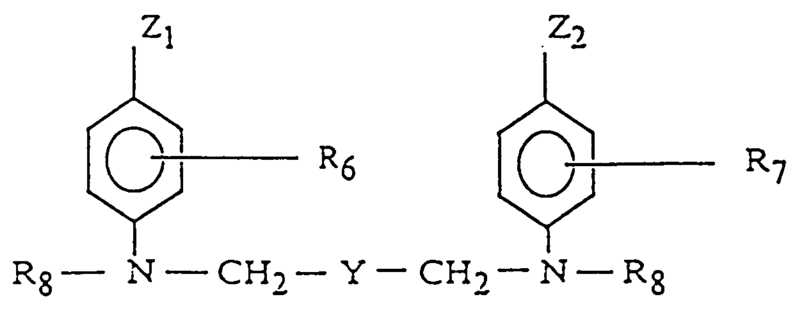

(III)

worin gilt:

$Z_1$ und $Z_2$ stellen, gleich oder verschieden, Hydroxyl- oder $NHR_9$-Gruppen dar, worin $R_9$ ein Wasserstoffatom oder eine Niedrigalkylrest bedeutet; $R_6$ und $R_7$ stellen, gleich oder verschieden, Wasserstoffatome, Halogenatome oder auch Alkylgruppen dar;

$R_8$ stellt ein Wasserstoffatom, eine Alkyl-, Hydroxyalkyl- oder Aminoalkylgruppe dar, deren Aminorest mit einer oder zwei Alkylgruppen substituiert sein kann;

Y stellt einen Rest dar, der aus den folgenden Resten ausgewählt ist:

$-(CH_2)_n-$, $(CH_2)_m-O-(CH_2)_m$,
$-(CH_2)_m-CHOH-(CH_2)_m$,

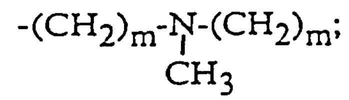

n ist eine ganze Zahl von 0 bis 8 und m eine ganze Zahl von 0 bis 4,

wobei diese doppelte genannten Basen auch in Form ihrer Additionssalze mit Säuren vorliegen können, und worin die oben aufgeführten Alkyl- oder Alkoxyreste eine Gruppe mit 1 bis 4 Kohlenstoffatomen bedeuten.

**10.** Färbezusammensetzung gemäß Anspruch 9, dadurch **gekennzeichnet**, daß die doppelte genannten Basen aus N,N'-Bis(β-hydroxyethyl)-N,N'-bis(4'-aminophenyl)-1,3-diaminopropan-2-ol, N,N'-Bis(β-hydroxyethyl)-N,N'-bis(4'-aminophenyl)ethylendiamin, N,N'-Bis(4-aminophenyl)-tetramethylendiamin, N,N'-Bis(β-hydroxyethyl)-N,N'-bis(4-aminophenyl)tetramethylendiamin, N,N'-Bis(4-methylaminophenyl)tetramethylendiamin, N,N'-Bis(ethyl)-N,N'-bis(4'-amino--3'-methylphenyl)-ethylendiamin ausgewählt sind.

**11.** Färbezusammensetzung gemäß einem der Ansprüche 1 bis 10, dadurch **gekennzeichnet**, daß sie auch weitere Kuppler enthält, die aus der Gruppe aus m-Diphenolen, m-Aminophenolen, m-Phenylendiaminen, m-Acylaminophenolen, m-Ureidophenolen, m-Carbalkoxyphenolen, α-Naphthol, Kupplern mit einer aktiven Methylengruppe, wie β-Ketoverbindungen, Pyrazolonen, heterozyklischen Kupplern und aus 4-, 6- und 7-Hydroxyindol ausgewählt sind.

**12.** Färbezusammensetzung gemäß Anspruch 11, dadurch **gekennzeichnet**, daß

sie Kuppler enthält, die aus der Gruppe aus insbesondere 2,4-Dihydroxyphenoxyethanol, 2,4-Dihydroxyanisol, m-Aminophenol, Monomethylether von Resorcin, 2-Methyl-5-N-(β-hyxdroxyethyl)-aminophenol, 2-Methyl-5-N-(β-mesylaminoethyl)aminophenol, 6-Hydroxybenzomorpholin, 2,4-Diaminoanisol, 2,4-Diaminophenoxyethanol, 6-Aminobenzomorpholin, (2-N-(β-Hydroxyethyl)amino-4-amino)-phenoxyethanol, 2-Amino-4-N-(β-hydroxyethyl)aminoanisol, (2,4-Diamino)phenyl-β-γ-dihydroxypropylether, 2,4-Diaminophenoxyethylamin, 1,3-Dimethoxy-2,4-diaminobenzol, 2-Methyl-5-aminophenol, 2,6-Dimethyl-3-aminophenol, 1-Amino-3,4-methylendioxybenzol, 1-Hydroxy-3,4-methylendioxybenzol, 2-Chlor-6-methyl-3-aminophenol, 2-Methyl-3-aminophenol, 2-Chlorresorcinol, Resorcinol, 6-Methoxy-3-hydroxyethylaminoanilin, 1-Ethoxy-2-bis(β-hydroxyethyl)amino-4-aminobenzol, 3-Diethylaminophenol, 1,3-Dihydroxy-2-methylbenzol, 1-Hydroxy-2,4-dichlor-3-aminobenzol, 4,6-Dihydroxyethoxy-1,3-diaminobenzol, 4-Methyl-6-ethoxy-1,3-diaminobenzol, 4-Chlor-6-methyl-3-aminophenol, 6-Chlor-3-trifluorethylaminophenol, aus deren Salzen und deren Mischungen ausgewählt sind.

**13.** Färbezusammensetzung gemäß jedem der Ansprüche 1 bis 12,
dadurch **gekennzeichnet**, daß
die Konzentration an Verbindungen der Formel (I) 0,05 bis 3,5 Gew.% des Gesamtgewichts der Zusammensetzung beträgt.

**14.** Färbezusammensetzung gemäß jedem der Ansprüche 1 bis 13,
dadurch **gekennzeichnet**, daß
die Gesamtkonzentration an Kupplern und an Oxidationsfarbstoff-Vorstufen vom para- und ortho-Typ 0,3 bis 7 Gew.% des Gesamtgewichts der Zuammensetzung beträgt.

**15.** Färbezusammensetzung gemäß jedem der Ansprüche 1 bis 14,
dadurch **gekennzeichnet**, daß
sie ausserdem Direktfarbstoffe enthält, ausgewählt aus Azo-, Anthrachinon-Farbstoffen und nitrierten Derivaten der Benzolreihe.

**16.** Färbezusammensetzung gemäß jedem der Ansprüche 1 bis 15,
dadurch **gekennzeichnet**, daß
sie einen pH-Wert von 4 bis 11 aufweist.

**17.** Färbezusammensetzung gemäß jedem der Ansprüche 1 bis 16,
dadurch **gekennzeichnet**, daß
sie 1 bis 40 Gew.% eines organischen Lösungsmittels enthält, das aus $C_{1-4}$-Niedrigalkanolen, Glycerin, Glycolen, Ethern von Glycol, aromatischen Alkoholen wie Benzylalkohol oder Phenoxyethanol und aus deren Mischungen ausgewählt ist.

**18.** Färbezusammensetzung gemäß jedem der Ansprüche 1 bis 17,
dadurch **gekennzeichnet**, daß
sie auch 0,5 bis 55 Gew.% eines oder mehrerer anionischer, kationischer, nicht-ionischer oder amphoterer oberflächenaktiver Mittel oder von deren Mischungen enthält.

**19.** Färbezusammensetzung gemäß jedem der Ansprüche 1 bis 18,
dadurch **gekennzeichnet**, daß
sie ausserdem kosmetische Hilfsstoffe enthält, ausgewählt aus Verdickungsmitteln, Antioxidantien, Eindringmitteln, Sequestriermitteln, Puffermitteln, Parfüm-Produkten, alkalisch stellenden Mitteln und aus Treibmitteln.

**20.** Färbezusammensetzung gemäß jedem der Ansprüche 1 bis 19,
dadurch **gekennzeichnet**, daß
sie in Form von Flüssigkeiten, Creme-Produkten, Gelen vorliegt oder in Form eines Aerosols in Gegenwart eines Treibmittels zubereitet ist.

**21.** Verfahren zur Färbung der Haare durch Oxidation,
dadurch **gekennzeichnet**, daß
man zum Zeitpunkt der Anwendung eine Färbezusammensetzung gemäß einem der Ansprüche 1 bis 20 mit einer oxidierenden Lösung in einer zur Oxidation des oder der Oxidationsfarbstoff-Vorstufen

ausreichenden Menge vermischt, wobei der pH der Mischung 3,5 bis 10 beträgt, und daß man dann die erhaltene Mischung auf die Haare aufbringt, sie 10 bis 40 Minuten lang verweilen läßt, dann die Haare spült, sie unter Schamponieren wäscht, erneut spült und trocknet.

**22.** Verfahren zur Färbung der Haare durch Oxidation, in mehreren Stufen,
dadurch **gekennzeichnet**, daß
man, in einer der Stufen, auf die Haare eine Färbezusammensetzung, enthaltend eine oder mehrere Oxidationsfarbstoff-Vorstufen vom para-Typ, ortho-Typ oder deren Mischungen, und dann in einer anderen Stufe eine Färbezusammensetzung aufträgt, die das Hydroxybenzofuran der Formel (I) enthält, wobei das oxidierende Mittel in der zum zweiten Zeitpunkt aufgetragenen Zusammensetzung vorhanden ist oder das oxidierende Mittel auch auf die Haare selbst zu einem dritten Zeitpunkt aufgebracht wird, und daß man das Ganze 10 bis 40 Minuten lang verweilen läßt, die Haare dann spült, sie unter Schamponieren wäscht, erneut spült und trocknet.